# EUROPÄISCHE PATENTANMELDUNG

(11) **EP 1 716 875 A2**
(43) Veröffentlichungstag der Anmeldung: **02.11.2006**
(21) Anmeldenummer: 06008536.2
(22) Anmeldetag: 25.04.2006
(51) Int. Cl.: A61M 1/16

(54) **Dialysekonzentrateinheit**

(30) Priorität: 26.04.2005 DE 202005006624 U
(71) Anmelder: B. Braun Medizintechnologie GmbH, 34212 Melsungen (DE)
(72) Erfinder: Wesseler, Matthias, 49326 Melle (DE); Renken, Martin, Dr., 01099 Dresden (DE); Hector, Rainer, Dr., 49082 Osnabrück (DE)
(74) Vertreter: Klingseisen, Franz

(57) **Zusammenfassung**

Es wird eine Dialysekonzentrateinheit vorgeschlagen, die einen Behälter (1) für wenigstens einen flüssigen Anteil der für das Dialysekonzentrat benötigten Rohstoffe und wenigstens einen weiteren Behälter (2) für die übrigen Rohstoffe in trockener Form umfasst, wobei diese Behälter (1, 2) in einem Aufnahmebehälter (4) zusammengefasst sind. Nach einer weiteren Ausführungsform wird Kochsalz in handelsüblichen Gebinden in trockener Form bereitgestellt, während alle anderen Komponenten der für das Dialysekonzentrat erforderlichen Rohstoffe in flüssiger Form in einem Behälter bereitgestellt werden und wobei Natriumchlorid in einem handelsüblichen Gebinde (5) in trockener Form und alle anderen Komponenten der für das Dialysekonzentrat erforderlichen Rohstoffe in flüssiger Form in einem Behälter (1) bereitgestellt werden, der auch den Anteil an Natriumchlorid in flüssiger Form enthält, der zu dem in trockener Form vorliegenden Anteil zusätzlich für das Dialysekonzentrat benötigt wird.

## Beschreibung

Für die Dialyse (künstliche Blutwäsche) wird Dialyseflüssigkeit benötigt, die dem Anwender z. B. Klinikpersonal, in Form eines Konzentrats zur Verfügung gestellt wird.

Die für ein Dialysekonzentrat benötigten Rohstoffe werden üblicherweise als Lösung bereitgestellt und verpackt, in der alle Rohstoffe gelöst sind. Hierdurch ergeben sich aufgrund des Gewichts und des Volumens schwer handhabbare Einheiten oder es werden kleinere Gebinde mit flüssigem Konzentrat bereitgestellt, wodurch aber der Herstellungs- und Transportaufwand vergrößert wird.

Rohstoffsets mit trockenen Komponenten werden angeboten, um gegenüber der herkömmlichen Verpackung von flüssigen Konzentraten in Kanistern, Beuteln oder Containern eine Gewichts- und Volumenersparnis zu erzielen. Beispielsweise enthalten 1001 Flüssigkonzentrat mit 1 g Glucose pro Liter fertiger Dialyselösung insgesamt bis zu 28 kg feste Substanzen, welche auch als solche abgepackt und zur bestimmungsgemäßen Verdünnung beim Anwender eingesetzt werden können. Zusätzlich enthalten 1001 Konzentrat ca. 0,63 kg flüssige Essigsäure.

Ein Beispiel für eine typische Rezeptur für 1001 Dialysekonzentrat umfasst:

| **Komponente** | **Menge für 100 l Konzentrat** |
|---|---|
| Natriumchlorid | 21,477 kg |
| Kaliumchlorid | 0,522 kg |
| Calciumchlorid-Dihydrat | 0,901 kg |
| Magnesiumchlorid-Hexahydrat | 0,712 kg |
| Glucosemonohydrat | 3,85 kg |
| Essigsäure (Eisessig) | 0,631 kg |

Werden die Komponenten einer solchen Rezeptur in trockenem Zustand gemischt für den Anwender bereitgestellt, so ergeben sich Probleme hinsichtlich Produktsicherheit und Qualitätskontrolle, weil das Gemenge aus den trockenen Komponenten nicht homogen ist und deshalb keine zuverlässigen Proben entnommen werden können, um die Qualität und die Zusammensetzung zu überprüfen.

Des Weiteren ist die einfache Beimengung der Essigsäure nicht möglich, weil diese stark flüchtig ist. Im Hinblick auf das Abpacken von Kalium-, Calcium- und Magnesiumchlorid ist auch die starke Neigung zum Verblocken und zur Aufnahme von Wasser aus der Umgebung zu berücksichtigen.

Die beschriebenen Probleme wurden bisher auf unterschiedliche Weise gelöst. Beispielsweise kann das Homogenisierungsproblem vermieden werden, indem alle Rohstoffe trocken und getrennt abgepackt werden und somit stets eine Kontrollmöglichkeit besteht. Die 630 g Eisessig müssen jedoch in flüssiger Form abgefüllt werden und werden aus Sicherheitsgründen auf unter 10%-ige Essigsäure verdünnt Dieses alles ist jedoch kostenintensiv und für den Anwender umständlich.

Weiterhin ist es bekannt, ein Dialyse-Trockenkonzentrat bereitzustellen, welches aus weitgehend homogenen, verfahrenstechnisch erzeugten Salzagglomerationen besteht, welche die Essigsäure aufgrund von Kapillareffekten relativ stabil in sich halten.

Aufgabe der Erfindung ist es, eine Dialysekonzentrateinheit so zu gestalten, dass die Herstellung einfach und eine zuverlässige Qualitätskontrolle möglich ist, während zugleich die Transportbedingungen verbessert werden durch geringes Volumen.

Erfindungsgemäß wird dies dadurch erreicht, dass ein Teil der Rohstoffe in flüssiger Form und der übrige Teil der Rohstoffe als Trockenkonzentrat bereitgestellt wird.

Bei einem Ausführungsbeispiel wird aus den Komponenten Kalium-, Calcium-, Magnesiumchlorid und Essigsäure eine hochkonzentrierte homogene Lösung chargenweise hergestellt. Für 1001 Dialysekonzentrat (bei einer Verdünnung von 1 Teil Konzentrat zu 34 Teilen Wasser) sind von dieser Lösung 81 erforderlich. Zusätzlich zu dieser Lösung wird NaCl und Glucose in trockener Form bereitgestellt. Durch diese Aufteilung in flüssigen und trockenen Anteil der Rohstoffe erreicht man eine Volumenverringerung der Verpackungseinheit auf ca. 1/3 des Volumens von herkömmlichem Flüssigkonzentrat. Zwar wiegt ein derartiges Rohstoffset bzw. eine entsprechende Verpackungseinheit ca. 40 kg, sodass es ca. 7 kg schwerer ist als die reinen Trockensubstanzen, jedoch ergibt die Aufteilung der Rohstoffe in einem festen und flüssigen Anteil günstige Produktionsbedingungen, lässt zuverlässige Qualitätskontrollen zu und vereinfacht den Transport wegen des geringeren Volumen, sodass diese Vorteile das gegenüber reinen Trockensubstanzen erhöhte Gewicht überwiegen.

Vorteilhafterweise werden die Komponenten mit geringem Gewichtsanteil sowie die Essigsäure in Wasser gelöst als Charge angesetzt und in Kanistern abgefüllt, wodurch der Produktionsaufwand und auch das Rückstellen und Überprüfen von Kontrollmustem erleichtert wird. Lediglich die mengenmäßig größeren Komponenten Kochsalz und Glucose werden in trockenem Zustand verpackt, wobei bevorzugt Einzelgebinde (z. B. Beutel) vorgesehen und zu einem Set platzsparend in einem Karton zusammengestellt werden.

Eine beispielshafte Ausführungsform einer Verpackungseinheit nach der Erfindung ist in der Zeichnung wiedergegeben. Es zeigen
- Fig. 1: eine Seitenansicht einer Verpackungseinheit,
- Fig. 2: eine Draufsicht, und
- Fig. 3: eine weitere Verpackungseinheit in einer Draufsicht.

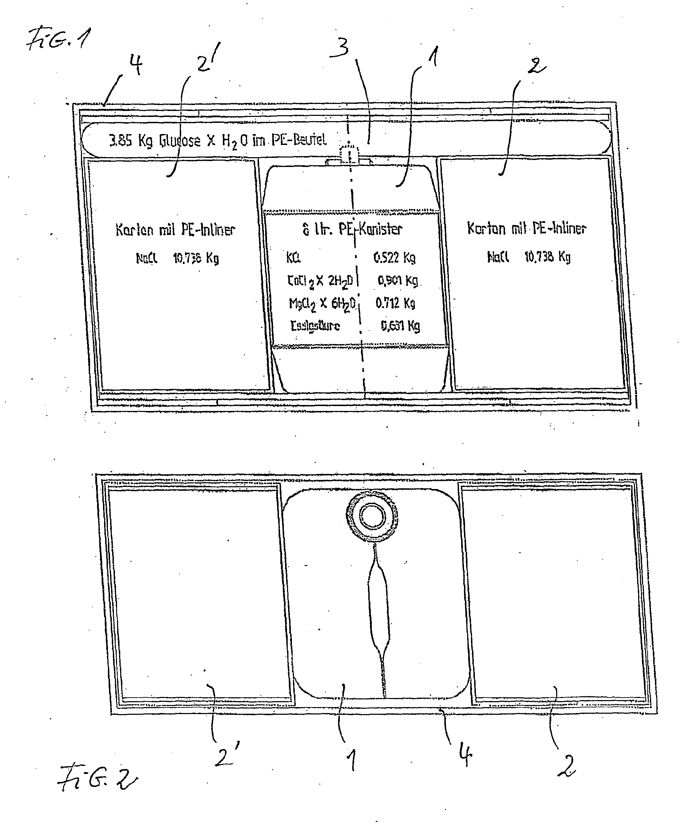

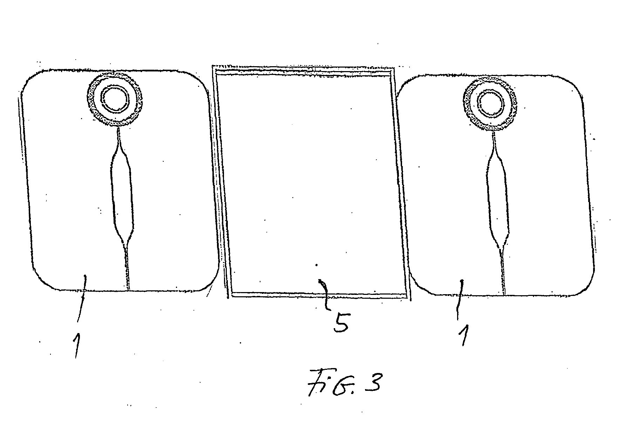

Mit 1 ist in Fig. 1 und 2 ein Kanister aus PE mit einem Inhaltsvolumen von 8 1 bezeichnet, der
0,522 kg KCl,
0,901 kg CaCl₂ x 2H₂O,
0,712 kg MgCl₂ x 6H₂O und
0,631 kg Essigsäure
enthält. Auf beiden Seiten des Kanisters 1 ist jeweils ein Karton 2 und 2' angeordnet, der mit PE ausgekleidet ist und jeweils 10,738 kg NaCl enthält. Über dem Kanister 1 und den beiden Kartons 2 und 2' ist ein flacher Beutel 3 aus PE positioniert, der 3,85 kg Glucose x H₂O enthält.

Mit 4 ist ein Umkarton mit eine Längenabmessung von 593 mm, einer Höhe von 338 mm und einer Breite von 263 mm bezeichnet, der die zuvor angegebenen Einheiten 1 bis 3 ohne Volumenverlust aufnimmt, wie die Seitenansicht in Fig. 1 und die Draufsicht in Fig. 2 ohne Beutel 3 zeigt.

Diese Verpackungseinheit ist besonders platzsparend und für den Transport beispielsweise auf Paletten gut geeignet, wobei die einzelnen Behälter 1, 2, 2' und 3 leicht manuell gehandhabt werden können. Gegenüber herkömmlichem Flüssigkonzentrat, bei dem sämtliche Rohstoffe in Lösung vorliegen, wird durch diese Verpackungseinheit eine Volumenreduktion auf ein Drittel erreicht. Gegenüber getrennt abgepackten trockenen Komponenten wird durch diese Verpackungseinheit eine Kostenersparnis bei der Bereitstellung erzielt und die Herstellung des Konzentrats beim Anwender vereinfacht.

Die obigen Gewichtsangaben beziehen sich auf ein Dialysekonzentrat (1 + 34) von 1001. Es ist auch möglich, entsprechend geringere Gewichtsanteile für eine Verpackungseinheit auf der Basis von 50 1 Dialysekonzentrat bereitzustellen, damit man kleinere Behälter 1, 2 mit geringerem Gewicht verwenden kann.

Die beiden Behälter 2 und 2' sind so ausgelegt, dass ihre Länge, Höhe und Breite den entsprechenden Abmessungen des Kanisters 1 entsprechen, sodass die drei Behälter 1, 2 und 2' zu einem Block zusammengestellt werden können, über dem der flache Beutel 3 mit Glucose angeordnet werden kann, dessen Länge und Breite der Länge und Breite dieses Blocks entsprechen. Zweckmäßigerweise wird ein etwa würfelförmiger Kanister oder Beutel 1 gewählt, auf dessen beiden Seiten etwa würfelförmige Behälter 2 angeordnet werden.

Nach einer anderen Ausgestaltung werden für den Anwender nur die flüssigen Komponenten bereitgestellt, sodass der Anwender selbst die trockenen Komponenten Kochsalz und Glucose aus marktüblichen Großgebinden mit Hilfe einer Waage hinzu dosieren kann, um fertiges Dialysekonzentrat zu erhalten.

Nach einer anderen Ausgestaltung wird für den Anwender die flüssige Komponente einschließlich Glucose in z. B. 10 1 Kanistern bereitgestellt, sodass der Anwender selbst durch Zugabe von Kochsalz aus marktüblichen Gebinden ein Dialysekonzentrat herstellen kann. So können aus 25 kg (marktübliches Gebinde) Kochsalz und zwei 101 Kanistern mit der flüssigen Komponente einschließlich Glucose mit Wasser 116,41 Dialysekonzentrat hergestellt werden. Werden zu der flüssigen Komponente noch zusätzlich 38,62 g/l Kochsalz hinzudosiert, so ergibt sich für den Dialysekonzentratansatz eine gerade Zahl von 120 Liter.

Insbesondere für den Transport größerer Mengen der für ein Dialysekonzentrat benötigten Rohstoffe z. B. auf einer Palette wird, wie Fig. 3 zeigt, Kochsalz in handelsüblichen Gebinden von beispielsweise 25 kg-Beuteln 5 in trockener Form bereitgestellt, während alle anderen Komponenten in flüssiger Form in einem oder zwei Kanistern 1 bereitgestellt werden, wobei die Kanister 1 auch den Anteil von Kochsalz in flüssiger Form enthalten, der zusätzlich zu dem in trockener Form in den Beuteln 5 vorliegenden Anteil für das Dialysekonzentrat benötigt wird.

Kochsalz in handelsüblichen Gebinden von beispielsweise 25 kg stellt die vom Kostenaufwand billigste Verpackung dar. Die zusätzlich für die Dialyseflüssigkeit benötigte Menge an Kochsalz wird in flüssiger Form im Kanister zusammen mit den anderen Komponenten bereitgestellt, sodass sich bei dieser Form der Verpackungseinheit nur handelsübliche Kochsalzgebinde in Verbindung mit einem oder zwei Kanistern ergeben. Diese Verpackungseinheiten sind insbesondere für den Transport größerer Mengen an Rohstoffen für Dialysekonzentrat auf Paletten von Vorteil. Bei diesen Verpackungseinheiten aus Kochsalz in handelsüblichen Gebinden von z. B. 25 kg und den übrigen Anteilen einschließlich des Restanteils an Kochsalz in flüssiger Form ist die Qualitätskontrolle einfach, weil nur der flüssige Inhalt der Kanister überprüft zu werden braucht. Der Anwender hat den Vorteil, dass er nur das in trockener Form vorliegende Kochsalz hinzuzugeben braucht.

## Patentansprüche

1. Dialysekonzentrateinheit, umfassend
einen Behälter (1) für wenigstens einen flüssigen Anteil der für das Dialysekonzentrat benötigten Rohstoffe und
wenigstens einen weiteren Behälter (2) für die übrigen Rohstoffe in trockener Form, wobei diese Behälter (1, 2) in einem Aufnahmebehälter (4)-zusammengefasst sind.

2. Dialysekonzentrateinheit nach Anspruch 1, wobei Rohstoffkomponenten mit geringem Gewichtsanteil in flüssiger Form verpackt sind und die mengenmäßig größeren Rohstoffkomponenten in trockener Form.

3. Dialysekonzentrateinheit nach Anspruch 1, wobei als Trockenkomponente NaCl in zwei Behälter (2, 2') von jeweils 10,738 kg aufgeteilt ist.

4. Dialysekonzentrateinheit nach Anspruch 4, wobei beiderseits eines Behälters (1) mit 8 1 Flüssigkomponenten jeweils ein Behälter (2, 2') mit etwa gleichen Abmessungen angeordnet ist und über diesen drei Behältern (2, 1, 2') ein flacher Beutel (3) mit 3,85 kg Glucose positioniert ist, dessen Länge und Breite der Länge und Breite der drei Behälter (2, 1, 2') entsprechen.

5. Dialysekonzentrateinheit nach Anspruch 4, wobei der die vier Behälter (1 bis 3) aufnehmende Umkarton (4) eine Länge von 593 mm, eine Höhe von 338 mm und eine Breite von 263 mm aufweist.

6. Dialysekonzentrateinheit für die für ein Dialysekonzentrat benötigten Rohstoffe, wobei in einem Behälter (1) nur jene Rohstoffkomponenten mit geringem Gewichtsanteil in flüssiger Form bereitgestellt werden.

7. Dialysekonzentrateinheit nach Anspruch 6, wobei in einem Behälter (1) die Komponenten
Natriumchlorid (anteilig)
Kaliumchlorid
Calciumchlorid-Dihydrat
Magnesiumchlorid-Hexahydrat und
Essigsäure (Eisessig)
in flüssiger Form bereitgestellt werden.

8. Dialysekonzentrateinheit nach Anspruch 7, wobei zusätzlich Glucosemonohydrat in dem Behälter (1) in flüssiger Form enthalten ist.

9. Dialysekonzentrateinheit, wobei Natriumchlorid in einem handelsüblichen Gebinde (5) in trockener Form und alle anderen Komponenten der für das Dialysekonzentrat erforderlichen Rohstoffe in flüssiger Form in einem Behälter (1) bereitgestellt werden, der auch den Anteil an Natriumchlorid in flüssiger Form enthält, der zu dem in trockener Form vorliegenden Anteil zusätzlich für das Dialysekonzentrat benötigt wird.
